# EUROPÄISCHE PATENTSCHRIFT

(11) **EP 1 723 103 B1**
(45) Veröffentlichungstag und Bekanntmachung des Hinweises auf die Patenterteilung: **14.01.2015**
(21) Anmeldenummer: 05733648.9
(22) Anmeldetag: 26.02.2005
(51) Int. Cl.: C07C 315/06, C07C 317/36

(54) **VERFAHREN ZUR AUFARBEITUNG VON 4-BETA-SULFATOETHYLSULFONYLANILIN-2-SULFONSAURE**
METHOD FOR PROCESSING 4-BETA-SULPHATOETHYLSULPHONYLANILINE-2-SULPHONIC ACID
PROCEDE DE PREPARATION D'ACIDE 4-BETA-SULFATOETHYLSULFONYLANILINE 2-SULFONIQUE

(30) Priorität: 03.03.2004 DE 102004010950
(43) Veröffentlichungstag der Anmeldung: 22.11.2006
(73) Patentinhaber: BASF SE, 67056 Ludwigshafen (DE)
(72) Erfinder: SOMOGYI, Laszlo, 67117 Limburgerhof (DE)
(86) Internationale Anmeldenummer: PCT/EP2005/002054
(87) Internationale Veröffentlichungsnummer: WO 2005/085190

(56) Entgegenhaltungen:
- EP-A- 0 753 509
- WO-A-96/02593
- DE-A1- 2 154 943
- DE-A1- 2 538 722
- DE-A1- 2 538 723

## Beschreibung

Die Erfindung betrifft ein Verfahren zur Aufarbeitung von 4-β-Sulfatoethylsulfonyl anilin-2-sulfonsäure das als Lösung in wässriger Schwefelsäure vorliegt.

β-Sulfatoethylsulfonylanilin-2-sulfonsäure wird häufig als Rohstoff zur Herstellung von Azofarbstoffen eingesetzt. Dazu muss dieser Rohstoff in hoher Reinheit vorliegen. Ein gängiges Verfahren zur Herstellung von β-Sulfatoethylsulfonylanilin-2-sulfonsäure ist aus DE-A 2 538 723 bekannt. Danach wird die entsprechende β-Sulfatoethylsulfonylanilin-2,6-disulfonsäure, die aus β-Sulfatoethylsulfonylanilin durch Behandlung mit Oleum erhältlich ist, in üblicherweise 30 bis 96.-%iger Schwefelsäure bei Temperaturen von im allgemeinen 80 bis 140 °C behandelt. Dabei wird ein Sulfonsäurerest abgespalten.

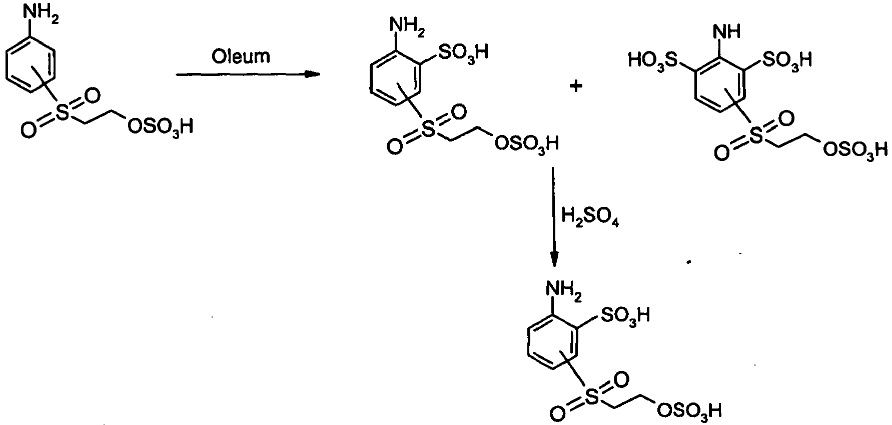

Anschließend wird die erhaltene Sulfierungsschmelze auf Eis gegeben.

Zur Aufarbeitung der β-Sulfatoethylsulfonylanilin-2-sulfonsäure aus dieser wässrigen schwefelsauren Lösung wird zunächst die überschüssige Schwefelsäure mit Alkali- oder Erdalkalihydroxiden bzw. -carbonaten neutralisiert. Aus dieser neutralen Lösung wird die Sulfatoethylsulfonylanilin-2-sulfonsäure durch Aussalzen mit Alkalihalogeniden oder durch Sprühtrocknen isoliert. In Beispiel 7 wird so gearbeitet, dass die Hauptmenge der überschüssigen Schwefelsäure mit Calciumcarbonat abgestumpft und der Rest mit Soda auf einen pH-Wert von 6 neutralisiert wird. Bei dieser Verfahrensweise ist jedoch die Produktreinheit verbesserungsbedürftig.

Es hat nicht an Versuchen gefehlt, das Aufarbeitungsverfahren zu verbessern. So wird in EP-A 753 509 offenbart, dass man aus der wässrigen schwefelsauren Lösung direkt die β-Sulfatoethylsulfonylanilin-2,6-disulfonsäure durch Zugabe von Kaliumchlorid ausfällen kann. Bei diesem Verfahren erhält man jedoch ein Produkt, das nicht lagerstabil ist und sich somit nur bedingt zur Herstellung von hochwertigen Azoreaktivfarbstoffen eignet. Die im Beispiel erwähnte Umkristallisation des ausgefällten Produktes führt weiterhin zu nicht unerheblichen Verlusten an Wertprodukt und ist damit unwirtschaftlich.

DE 2154943 offenbart ein Verfahren zur Aufarbeitung von einer Lösung von 4-(β-Sulfatoethylsulfonyl)anilin-2-sulfonsäure in wässriger Schwefelsäure durch Zugabe von calciniertem Natriumcarbonat auf einen pH-Wert von 6,0 bis 6,5.

WO 96/02593 offenbart ein Verfahren zur Herstellung von 5-(β-Sulfatoethylsulfonyl)anilin-2-sulfonsäure, wobei das Produkt von einer wässrigen Lösung durch Zugabe von calciniertem Soda bei einem pH-Wert von 4,5 bis 5 ausfällt.

Es bestand daher die Aufgabe, ein Verfahren bereitzustellen, mit dem in technisch einfacher Weise 4-β-Sulfatoethylsulfonylanilin-2-sulfonsäure aus schwefelsaurer wässriger Lösung als lagestabiles, reines Produkt ohne wesentliche Verluste hergestellt werden kann.

Demgemäss wurde ein Verfahren zur Aufarbeitung von 4-β-Sulfatoethylsulfonylanilin-2-sulfonsäure das als Lösung in wässriger Schwefelsäure vorliegt gefunden, das dadurch gekennzeichnet ist, dass man
- in einem ersten Schritt das 4-β-Sulfatoethylsulfonylanilin-2-sulfonsäure durch Zugabe von (Erd)alkali- und/oder Ammoniumsalzen ausfällt, abtrennt und in Wasser löst, oder die Schwefelsäure durch Zugabe von Calciumsalzen ausfällt, das entstehende Calciumsulfat gegebenenfalls abtrennt, und
- in einem zweiten Schritt die entstandene Lösung durch Zugabe von (Erd-)alkali- oder Ammoniumhydroxid, -carbonat, -hydrogencarbonat und/oder -acetat auf einen pH-Wert zwischen 1 und 5 einstellt, noch vorhandenes Calciumsulfat abtrennt, und die 4-β-Sulfatoethylsulfonylanilin-2-sulfonsäure aus der Lösung isoliert.

Weiterhin wurde die so herstellbare 4-β-Sulfatoethylsulfonylanilin-2-sulfonsäure gefunden.

Unter β-Sulfatoethylsulfonylanilin-2-sulfonsäure wird im Sinne dieser Patentanmeldung nur die 4-β-Sulfatoethylsulfonylanilin-2-sulfonsäure verstanden, und zwar sowohl die freie Säure als auch ihre Salzformen wie Mono- oder Di-Alkali-, Mono- oder Di-Ammonium- bzw. Erdalkalisalze mit gleichen oder verschiedenen Gegenionen.

Das erfindungsgemäße Verfahren setzt an bei einer wässrigen schwefelsauren Lösung von β-Sulfatoethylsulfonylanilin-2-sulfonsäure mit einer Konzentration von im allgemeinen 1 bis 30 Gew.-%, in vielen Fällen 1 bis 20 Gew.-%, insbesondere 5 bis 20 Gew.-% β-Sulfatoethylsulfonylanilin-2-sulfonsäure und üblicherweise 1 bis 80, in vielen Fällen 5 bis 50 Gew.-% Schwefelsäure. Solche Lösungen sind beispielsweise durch die Sulfierung von β-Sulfatoethylsulfonylanilin oder durch die Desulfierung von β-Sulfatoethylsulfonylanilin-2,6-disulfonsäure erhältlich.

Der erste Schritt dient dazu, die β-Sulfatoethylsulfonylanilin-2-sulfonsäure von der Schwefelsäure zu trennen.

Zur Ausfällung der β-Sulfatoethylsulfonylanilin-2-sulfonsäure kommen Erdalkali-, Alkali-, Ammoniumsalze oder Mischungen daraus, vorzugsweise Natrium-, Kalium- und/oder Ammoniumsalze, insbesondere Natriumchlorid, Natriumsulfat, Kaliumchlorid, Kaliumsulfat oder Mischungen daraus in Frage. Die Mengen können in weiten Bereichen variiert werden, üblicherweise verwendet man 1 bis 10 , insbesondere 2 bis 6 Moläquivalente. Die Produktabtrennung ist nicht kritisch, sie kann mit üblichen Apparaten wie Filterpressen, Rührdrucknutschen, Ultrafiltrationsanlagen oder Bandfiltern durchgeführt werden.

Das nachfolgende Auflösen des abgetrennten Produktes in Wasser ist ebenfalls unkritisch, es kann bei Raumtemperatur, bevorzugt jedoch unter Kühlung bei 0 bis 20°C oder am einfachsten durch Eintragen in üblicherweise 1 bis 20 Liter, in vielen Fällen 1 bis 10 Liter Eiswasser pro Mol Produkt ausgeführt werden.

Es ist ebenfalls möglich, im ersten Schritt die Schwefelsäure durch Zugabe von Calciumsalzen wie Calciumcarbonat und/oder Calciumoxid auszufällen. Üblicherweise setzt man solche Mengen an Calciumsalzen ein, dass der pH-Wert sich auf 0 bis 2 einstellt.

Die Abtrennung des Calciumsulfates ist nicht kritisch, sie kann im ersten Schritt erfolgen oder im zweiten Schritt nach der Einstellung des gewünschten pH-Wertes. Üblicherweise werden Apparaten wie Filterpressen, Rührdrucknutschen, Ultrafiltrationsanlagen oder Bandfiltern eingesetzt.

Diese Lösung wird im zweiten Schritt durch Zugabe von Alkali-, Erdalkali- oder Ammoniumhydroxid, -carbonat, -hydrogencarbonat, -acetat oder Mischungen daraus, insbesondere Alkalicarbonat und/oder Alkalihydrogencarbonat, beispielsweise Soda oder Natriumhydrogencarbonat auf einen pH-Wert von 1 bis 5, insbesondere 2 bis 4, eingestellt.

Die anschließende Isolierung der β-Sulfatoethylsulfonylanilin-2-sulfonsäure ist nicht kritisch und kann durch Ausfällen, Eindampfen oder Sprühtrocknung geschehen. Die Ausfällung und die Isolierung des Produktes kann wie oben beschrieben gestaltet werden. Das Eindampfen geschieht im allgemeinen in bekannten Vakuumtrockenschränken bei einer Temperatur von 30 bis 70, insbesondere 35 bis 55 °C, die in vielen Fällen vorteilhafte Sprühtrocknung in üblichen Sprühtrocknungstürmen.

Das Verfahren hat viele Vorteile. Man spart große Mengen an Nebenprodukten und Abwärme und erhält in hoher Ausbeute ein reines Produkt mit ausgewogenen Verwendungseigenschaften.

### Beispiele:

### Vergleichsversuch 1 (EP 753 509)

In eine Vorlage aus 437 g 20.-%igem Oleum werden bei Raumtemperatur beginnend 142 g = 0,485 Mol 4-β-Sulfatoethylsulfonyl-anilin eingetragen; wobei die Temperatur auf 50°C ansteigt. Anschließend erwärmt man die Lösung 3 Stunden auf 115 ±2°C. Die Sulfierung wird auf 70 bis 80°C abgekühlt. Man tropft dann 42 g 50 gew.-%ige Schwefelsäure ein, wobei die Temperatur nicht über 100°C ansteigen soll. Anschließend erwärmt man zehn Stunden auf 95 bis 100°C.

Die Schmelze wird auf 40 bis 50°C abgekühlt und unter Kühlung von außen auf ein Gemisch von 700g Eiswasser gegeben. Dabei soll die Temperatur nicht über 20°C ansteigen. Zu der resultierenden klaren Lösung werden 75 g = 1,0 Mol Kaliumchlorid gegeben. Man rührt drei Stunden nach und saugt das ausgeschiedene Reaktionsprodukt ab.

Das Produkt wird durch Umkristallisation gereinigt: in 200 ml Wasser suspendiert, auf 70°C geheizt, dann in 100 ml-Portionen 500 ml 70°C heißes Wasser zugegeben bis alles gelöst ist. Die Mischung wird mit Eisbad auf 0 - 5° abgekühlt, der ausgefallene Rückstand abgesaugt und getrocknet. Der Rückstand wiegt 93,6 g und ist 79,2.-%ig bezogen auf Mol 361 4-β-Sulfatoethylsulfonylanilin-2-sulfonsäure. Ausbeute: 41%

### Beispiel 2

In eine Vorlage aus 437 g 20.-%igem Oleum werden bei Raumtemperatur beginnend 142 g = 0,485 Mol 4-β-Sulfatoethylsulfonyl-anilin eingetragen; wobei die Temperatur auf 50°C ansteigt. Anschließend erwärmt man die Lösung 3 Stunden auf 115 ±2°C. Die Sulfierung wird auf 70 bis 80 °C abgekühlt. Man tropft dann 42 g 50 gew.-%ige Schwefelsäure ein, wobei die Temperatur nicht über 100°C ansteigen soll. Anschließend erwärmt man zehn Stunden auf 95 bis 100°C.

Die Schmelze wird auf 40 bis 50°C abgekühlt und unter Kühlung von außen auf ein Gemisch von 700g Eiswasser gegeben. Dabei soll die Temperatur nicht über 20°C ansteigen. Zu der resultierenden klaren Lösung werden 33,6 g = 0,45 mol Kaliumchlorid zugegeben, gefolgt mit der Zugabe von drei mal 0,833 mol (48,7 g) Natriumchlorid, jeweils eine halbe Stunde nachgerührt. Das ausgefallene Produkt wird abgesaugt und in 1200 ml Wasser bei 15°C gelöst. Der pH-Wert der Lösung wird mit portionsweise zugegebenem Soda auf 3,0 eingestellt. Die Reaktionslösung wird bei 50°C im Vakuum zur Trockene eingeengt. Der Rückstand wiegt 412 g und ist 38.-%ig bezogen auf Mol 361 4-β-Sulfatoethylsulfonylanilin-2-sulfonsäure. Ausbeute: 87%

### Beispiel 3

4 Mol (1171 g) 4-β-Sulfatoethylsulfonyl-anilin (96.-%ig) wird in 2100 g 96 gew.-%ige Schwefelsäure eingetragen, wobei die Temperatur auf 35°C steigt. Anschließend gibt man 1660 g Oleum 65% zu, wobei die Temperatur auf 80°C steigt. Die Mischung wird auf 115°C geheizt und 3 Stunden bei 115°C gerührt. Nach Abkühlen auf 65°C wird 175 g Eis zugegeben, wobei die Temperatur auf 90°C steigt. Die Reaktionsmischung wird auf 95°C geheizt und 10 Stunden bei 95°C gerührt. 5800 g Eis wird vorgelegt und die auf Raumtemperatur abgekühlte Reaktionsmischung unter Eisbadkühlung so zulaufen lassen, dass die Temperatur nicht über 20°C steigt. Anschließend wird drei mal 5,128 mol (300 g) Natriumchlorid zugegeben und jeweils eine Stunde nachgerührt. Das ausgefallene Produkt wird abgesaugt und in Eiswasser (1538 g Eis; 7600 ml Wasser) bei 12°C gelöst. Der pH-Wert der Lösung wird mit portionsweise zugegebenem Soda auf 3,0 eingestellt. Die Temperatur steigt dabei auf 24°C. Die Lösung wird über Nacht bei Raumtemperatur gerührt und am nächsten Tag sprühgetrocknet. Der Rückstand wiegt 2155 g und ist 47.-%ig bezogen auf Mol 361 4-β-Sulfatoethylsulfonylanilin-2-sulfonsäure. Ausbeute: 70 %

### Beispiel 4

4 Mol (1171 g) 4-β-Sulfatoethylsulfonyl-anilin (96.-%ig) wird in 2100 g 96 gew.-%ige Schwefelsäure eingetragen; wobei die Temperatur auf 35°C steigt. Anschließend gibt man 1660 g Oleum 65% zu, wobei die Temperatur auf 80°C steigt. Die Mischung wird auf 115°C geheizt und 3 Stunden bei 115°C gerührt. Nach Abkühlen auf 65°C wird 175 g Eis zugegeben, wobei die Temperatur auf 90°C steigt. Die Reaktionsmischung wird auf 95°C geheizt und 10 Stunden bei 95°C gerührt. 5800 g Eis wird vorgelegt und die auf Raumtemperatur abgekühlte Reaktionsmischung unter Eisbadkühlung so zulaufen lassen, dass die Temperatur nicht über 20°C steigt. Anschließend wird drei mal

5,128 mol (300 g) Natriumchlorid zugegeben und jeweils eine Stunde nachgerührt. Das ausgefallene Produkt wird abgesaugt und in Eiswasser (1538 g Eis; 7600 ml Wasser) bei 12°C gelöst. Der pH-Wert der Lösung wird mit portionsweise zugegebenem Soda auf 3,0 eingestellt. Die Temperatur steigt dabei auf 22°C. Die Lösung wird übers Wochenende bei Raumtemperatur gerührt und anschließend wird 1500 g NaCl zugegeben, wodurch das Produkt ausgesalzen wird. Nach Absaugen und Trocknen wiegt der Rückstand 2094,8 g und ist 52,5.-%ig bezogen auf Mol 361 4-β-Sulfatoethylsulfonylanilin-2-sulfonsäure. Ausbeute: 76 %

### Beispiel 5

Zu einer Mischung aus 1 mol (361 g) 4-β-Sulfatoethylsulfonylanilin-2-sulfonsäure, 542 g Schwefelsäure und 386 g Wasser wird 2 mol (150 g) KCl in drei Portionen zugegeben und jeweils eine Stunde nachgerührt. Dabei fällt die 4-β-Sulfatoethylsulfonylanilin-2-sulfonsäure als di-Kaliumsalz aus. Das ausgefallene Produkt wird abgesaugt und in 2600 g Eiswasser bei 15°C suspendiert. Der pH-Wert der Mischung wird durch langsame Zugabe von 10 Gew.-%iger NaOH Lösung auf 5 eingestellt, wobei die Temperatur auf 25°C steigt. Anschließend wird die Lösung ultrafiltriert und das Produkt durch Sprühtrocknung isoliert.

### Beispiel 6

Zu einer Mischung aus 1 mol (361 g) 4-β-Sulfatoethylsulfonylanilin-2-sulfonsäure, 1125 g Schwefelsäure und 3014 g Wasser wird 9 mol (531 g) NaCl in drei Portionen zugegeben und jeweils eine Stunde nachgerührt. Dabei fällt die 4-β-Sulfatoethylsulfonylanilin-2-sulfonsäure als di-Natriumsalz aus. Das ausgefallene Produkt wird abgesaugt und in 3300 g Eiswasser bei 12°C suspendiert. Der pH-Wert der Mischung wird durch langsame Zugabe von 10 Gew.-%iger NaOH Lösung auf 4 eingestellt, wobei die Temperatur auf 23°C steigt. Anschließend wird die Lösung eingedampft.

### Beispiel 7

Zu einer Mischung aus 2 mol (722 g) 4-β-Sulfatoethylsulfonylanilin-2-sulfonsäure, 10100 g Schwefelsäure und 3600 g Wasser wird 5 mol (710 g) Na₂SO₄ in drei Portionen zugegeben und jeweils eine Stunde nachgerührt. Dabei fällt die 4-β-Sulfatoethylsulfonylanilin-2-sulfonsäure als di-Natriumsalz aus. Das ausgefallene Produkt wird abgesaugt und in 18000 g Eiswasser bei 12°C suspendiert. Der pH-Wert der Mischung wird durch langsame Zugabe von K₂CO₃ auf 3 eingestellt, wobei die Temperatur auf 25°C steigt. Anschließend wird die Lösung sprühgetrocknet.

### Beispiel 8

Zu einer Mischung aus 1 mol (361 g) 4-β-Sulfatoethylsulfonylanilin-2-sulfonsäure, 988 g Schwefelsäure und 1250 g Wasser wird 4 mol (696 g) K₂SO₄ in drei Portionen zugegeben und jeweils eine Stunde nachgerührt. Dabei fällt die 4-β-Sulfatoethylsulfonylanilin-2-sulfonsäure als di-Kaliumsalz aus. Das ausgefallene Produkt wird abgesaugt und in 18000 g Eiswasser bei 8°C suspendiert. Der pH-Wert der Mischung wird durch langsame Zugabe von KHCO₃ auf 2 eingestellt, wobei die Temperatur auf 15°C steigt. Anschließend wird das Produkt durch Zugabe von 950 g KCl ausgesalzen, abgesaugt und getrocknet.

### Beispiel 9

Zu einer Mischung aus 1 mol (361 g) 4-β-Sulfatoethylsulfonylanilin-2-sulfonsäure, 1080 g Schwefelsäure und 809 g Wasser wird 6 mol (354 g) NaCl in drei Portionen zugegeben und jeweils eine Stunde nachgerührt. Dabei fällt die 4-β-Sulfatoethylsulfonylanilin-2-sulfonsäure als di-Natriumsalz aus. Das ausgefallene Produkt wird abgesaugt und in 4200 g Eiswasser bei 14°C suspendiert. Der pH-Wert der Mischung wird durch langsame Zugabe von NaHCO₃ auf 1 eingestellt, wobei die Temperatur auf 22°C steigt. Anschließend wird das Produkt durch Zugabe von 882 g NaCl ausgesalzen, abgesaugt und getrocknet.

### Beispiel 10

Zu einer Mischung aus 1 mol (361 g) 4-β-Sulfatoethylsulfonylanilin-2-sulfonsäure, 1580 g Schwefelsäure und 5260 g Wasser wird 1 mol (174 g) K₂SO₄ und 4 mol (568 g) Na₂SO₄ zugegeben und jeweils eine Stunde nachgerührt. Dabei fällt die 4-β-Sulfatoethylsulfonylanilin-2-sulfonsäure aus. Das ausgefallene Produkt wird abgesaugt und in 4200 g Eiswasser bei 15°C suspendiert. Der pH-Wert der Mischung wird durch langsame Zugabe von Na-Acetat auf 3 eingestellt, wobei die Temperatur auf 25°C steigt. Anschließend wird die Lösung sprühgetrocknet.

### Beispiel 11

Zu einer Mischung aus 1 mol (361 g) 4-β-Sulfatoethylsulfonylanilin-2-sulfonsäure, 864 g Schwefelsäure und 6000 g Wasser wird 650 g CaCO₃ zugegeben gefolgt mit der Zugabe von Soda, bis der pH-Wert 3 beträgt. Die Temperatur wird unter 20°C gehalten. Das ausgefallene Calciumsulfathalbhydrat wird abgesaugt und die 4-β-Sulfatoethylsulfonylanilin-2-sulfonsäure als di-Natriumsalz durch Sprühtrocknung der Filtratlösung isoliert.

### Beispiel 12

Zu einer Mischung aus 1 mol (361 g) 4-β-Sulfatoethylsulfonylanilin-2-sulfonsäure, 900 g Schwefelsäure und 16700 g Wasser wird 680 g CaCO₃ zugegeben gefolgt mit der Zugabe von K₂CO₃, bis der pH-Wert 1 beträgt. Die Temperatur wird unter 60°C gehalten. Das ausgefallene Calciumsulfathalbhydrat wird abgesaugt und die 4-β-Sulfatoethylsulfonylanilin-2-sulfonsäure als di-Kaliumsalz durch Eindampfen der Filtratlösung isoliert.

### Beispiel 13

Zu einer Mischung aus 1 mol (361 g) 4-β-Sulfatoethylsulfonylanilin-2-sulfonsäure, 860 g Schwefelsäure und 5800 g Wasser wird 650 g CaCO₃ zugegeben gefolgt mit der Zugabe von NaHCO₃, bis der pH-Wert 2 beträgt. Die Temperatur wird unter 40°C gehalten. Das ausgefallene Calciumsulfathalbhydrat wird abgesaugt und die 4-β-Sulfatoethylsulfonylanilin-2-sulfonsäure als di-Natriumsalz durch Sprühtrocknung der Filtratlösung isoliert.

### Beispiel 14

Zu einer Mischung aus 0,5 mol (180,5 g) 4-β-Sulfatoethylsulfonylanilin-2-sulfonsäure, 433 g Schwefelsäure und 1200 g Wasser wird 350 g CaCO₃ zugegeben gefolgt mit der Zugabe von KHCO₃, bis der pH-Wert 4 beträgt. Die Temperatur wird unter 35°C gehalten. Das ausgefallene Calciumsulfathalbhydrat wird abgesaugt und die 4-β-Sulfatoethylsulfonylanilin-2-sulfonsäure durch Zugabe von 680 g KCl ausgesalzen, abgesaugt und getrocknet.

### Beispiel 15

Zu einer Mischung aus 2 mol (722 g) 4-β-Sulfatoethylsulfonylanilin-2-sulfonsäure, 1680 g Schwefelsäure und 2400 g Wasser wird 1300 g CaCO₃ zugegeben gefolgt mit der Zugabe von 10 Gew.-%iger NaOH Lösung, bis der pH-Wert 5 beträgt. Die Temperatur wird unter 25°C gehalten. Das ausgefallene Calciumsulfathalbhydrat wird abgesaugt und die 4-β-Sulfatoethylsulfonylanilin-2-sulfonsäure durch Zugabe von 1450 g NaCl ausgesalzen, abgesaugt und getrocknet.

### Beispiel 16

Zu einer Mischung aus 1 mol (361 g) 4-β-Sulfatoethylsulfonylanilin-2-sulfonsäure, 871 g Schwefelsäure und 6100 g Wasser wird 740 g CaCO₃ zugegeben. Das ausgefallene Calciumsulfathalbhydrat wird abgesaugt und der pH-Wert der Filtratlösung wird durch der Zugabe von Na-Acetat auf 3 angehoben. Die Temperatur wird unter 20°C gehalten. Die 4-β-Sulfatoethylsulfonylanilin-2-sulfonsäure wird als di-Natriumsalz durch Sprühtrocknung der Filtratlösung isoliert.

### Beispiel 17

Zu einer Mischung aus 1 mol (361 g) 4-β-Sulfatoethylsulfonylanilin-2-sulfonsäure, 885 g Schwefelsäure und 2350 g Wasser wird 725 g CaCO₃ zugegeben. Das ausgefallene Calciumsulfathalbhydrat wird abgesaugt und der pH-Wert der Filtratlösung wird durch der Zugabe von Soda auf 4 angehoben. Die Temperatur wird unter 28°C gehalten. Die 4-β-Sulfatoethylsulfonylanilin-2-sulfonsäure wird als di-Natriumsalz durch Eindampfen der Filtratlösung isoiiert.

### Beispiel 18

Zu einer Mischung aus 1 mol (361 g) 4-β-Sulfatoethylsulfonylanilin-2-sulfonsäure, 885 g Schwefelsäure und 2350 g Wasser wird bei 25°C CaCO₃ zugegeben, bis der pH-Wert auf 3 eingestellt ist. Das ausgefallene Calciumsulfathalbhydrat wird abgesaugt und die 4-β-Sulfatoethylsulfonylanilin-2-sulfonsäure wird als Calciumsalz durch Sprühtrocknung der Filtratlösung isoliert.

## Patentansprüche

1. Verfahren zur Aufarbeitung von 4-β-Sulfatoethylsulfonylanilin-2-sulfonsäure, das als Lösung in wässriger Schwefelsäure vorliegt, **dadurch gekennzeichnet, dass** man
in einem ersten Schritt die 4-β-Sulfatoethylsulfonylanilin-2-sulfonsäure durch Zugabe von (Erd)alkali- und/oder Ammoniumsalzen ausfällt, abtrennt und in Wasser löst, oder die Schwefelsäure durch Zugabe von Calciumsalzen ausfällt, das entstehende Calciumsulfat gegebenenfalls abtrennt, und
in einem zweiten Schritt die entstandene Lösung durch Zugabe von (Erd-)alkali- oder Ammoniumhydroxid, -carbonat, -hydrogencarbonat und/oder -acetat auf einen pH-Wert zwischen 1 und 5 einstellt, noch vorhandenes Calciumsulfat abtrennt, und die 4-β-Sulfatoethylsulfonylanilin-2-sulfonsäure aus der Lösung isoliert.

2. Verfahren nach Anspruch 1, **dadurch gekennzeichnet, dass** man im ersten Schritt die 4-β-Sulfatoethylsulfonylanilin-2-sulfonsäure durch Zugabe von 1 bis 10 Moläquivalenten Natrium-, Kalium- und/oder Ammoniumsalz ausfällt.

3. Verfahren nach einem der Ansprüche 1 oder 2, **dadurch gekennzeichnet, dass** man im ersten Schritt als Salz Natriumchlorid, Natriumsulfat, Kaliumchlorid oder Kaliumsulfat einsetzt.

4. Verfahren nach einem der Ansprüche 1 bis 3, **dadurch gekennzeichnet, dass** man im zweiten Schritt die 4-β-Sulfatoethylsulfonylanilin-2-sulfonsäure durch Ausfällen mit Natrium-, Kalium- und/oder Ammoniumsalzen oder durch Sprühtrocknen isoliert.

5. Verfahren nach Anspruch 4, **dadurch gekennzeichnet, dass** man im zweiten Schritt die 4-β-Sulfatoethylsulfonylanilin-2-sulfonsäure durch Ausfällen mit 1 bis 10 Moläquivalenten der Salze isoliert.

6. Verfahren nach Anspruch 4 oder 5, **dadurch gekennzeichnet, dass** man als Natriumsalz Natriumchlorid oder Natriumsulfat und als Kaliumsalz Kaliumchlorid oder Kaliumsulfat einsetzt.

## Claims

1. A process for working up 4-β-sulfatoethylsulfonylaniline-2-sulfonic acid from a solution in aqueous sulfuric acid, which comprises a first step of the 4-β-sulfatoethylsulfonylaniline-2-sulfonic acid being precipitated by addition of alkali or alkaline earth metal and/or ammonium salts, separated off and dissolved in water or the sulfuric acid being precipitated by addition of calcium salts and optionally the resulting calcium sulfate being separated off, and a second step of the resultant solution being adjusted to a pH between 1 and 5 by addition of alkali or alkaline earth metal or ammonium hydroxide, carbonate, bicarbonate and/or acetate, any calcium sulfate still present being separated off and the 4-β-sulfatoethylsulfonylaniline-2-sulfonic acid being isolated from the solution.

2. The process according to claim 1 wherein the 4-β-sulfatoethylsulfonylaniline-2-sulfonic acid is precipitated by addition of 1 to 10 mol equivalents of sodium, potassium and/or ammonium salt in the first step.

3. The process according to claim 1 or claim 2 that utilizes sodium chloride, sodium sulfate, potassium chloride or potassium sulfate salt in the first step.

4. The process according to any of claims 1 to 3 wherein the 4-β-sulfatoethylsulfonylaniline-2-sulfonic acid is isolated in the second step by precipitating with sodium, potassium and/or ammonium salts or by spray drying.

5. The process according to claim 4 wherein the 4-β-sulfatoethylsulfonylaniline-2-sulfonic acid is isolated in the second step by precipitating with 1 to 10 mol equivalents of the salts.

6. The process according to claim 4 or 5 that utilizes sodium chloride or sodium sulfate as sodium salt and potassium chloride or potassium sulfate as potassium salt.

## Revendications

1. Procédé pour le traitement d'acide 4-ß-sulfatoéthylsulfonylaniline-2-sulfonique, qui se trouve sous forme de solution dans de l'acide sulfurique aqueux, **caractérisé en ce que**, dans une première étape, on précipite l'acide 4-ß-sulfatoéthylsulfonylaniline-2-sulfonique par addition de sels de métal alcalin ou alcalino-terreux et/ou d'ammonium, on le sépare et on le dissout dans l'eau, ou on précipite l'acide sulfurique par addition de sels de calcium, on sépare le cas échéant le sulfate de calcium qui se forme et dans une deuxième étape, on règle la solution formée à un pH entre 1 et 5 par addition d'hydroxyde, de carbonate, d'hydrogénocarbonate et/ou d'acétate de métal alcalin ou alcalino-terreux ou d'ammonium, on sépare le sulfate de calcium encore présent et on isole l'acide 4-ß-sulfatoéthylsulfonylaniline-2-sulfonique de la solution.

2. Procédé selon la revendication 1, **caractérisé en ce que**, dans la première étape, on précipite l'acide 4-ß-sufatoéthylsulfonylaniline-2-sulfonique par addition de 1 à 10 équivalents en mole de sel de sodium, de potassium et/ou d'ammonium.

3. Procédé selon l'une quelconque des revendications 1 ou 2, **caractérisé en ce que**, dans la première étape, on utilise, comme sel, du chlorure de sodium, du sulfate de sodium, du chlorure de potassium ou du sulfate de potassium.

4. Procédé selon l'une quelconque des revendications 1 à 3, **caractérisé en ce que**, dans la deuxième étape, on isole l'acide 4-ß-sulfatoéthylsulfonylaniline-2-sulfonique par précipitation à l'aide de sels de sodium, de potassium et/ou d'ammonium ou par séchage par pulvérisation.

5. Procédé selon la revendication 4, **caractérisé en ce que**, dans la deuxième étape, on isole l'acide 4-ß-sulfatoéthylsulfonylaniline-2-sulfonique par précipitation avec 1 à 10 équivalents en mole des sels.

6. Procédé selon la revendication 4 ou 5, **caractérisé en ce qu'**on utilise, comme sel de sodium, du chlorure de sodium ou du sulfate de sodium et, comme sel de potassium, du chlorure de potassium ou du sulfate de potassium.
